# EUROPEAN PATENT SPECIFICATION

(11) **EP 2 873 413 B1**
(45) Date of publication and mention of the grant of the patent: **05.09.2018**
(21) Application number: 13816982.6
(22) Date of filing: 10.07.2013
(51) Int. Cl.: A61K 8/63, A61K 8/06, A61K 8/891, A61Q 19/00, A61K 8/58, A61Q 5/12

(54) **WATER-IN-OIL EMULSION COSMETIC**
KOSMETISCHE WASSER-IN-ÖL-EMULSION
PRODUIT COSMÉTIQUE D'ÉMULSION EAU-DANS-HUILE

(30) Priority: 10.07.2012 JP 2012154632
(43) Date of publication of application: 20.05.2015
(73) Proprietor: Shiseido Company, Ltd., Tokyo 104-0061 (JP)
(72) Inventor: KITAJIMA, Masaki, Yokohama-shi Kanagawa 224-8558 (JP); IBE, Ayako, Yokohama-shi Kanagawa 224-8558 (JP); WATANABE, Kei, Yokohama-shi Kanagawa 224-8558 (JP)
(74) Representative: Henkel, Breuer & Partner
(86) International application number: PCT/JP2013/068848
(87) International publication number: WO 2014/010622

(56) References cited:
- WO-A2-2011/065772
- JP-A- H0 570 335
- JP-A- 2002 255 738
- JP-A- 2005 104 854
- JP-A- 2008 013 515

## Description

### [Related Applications]

This application claims the priority of Japanese Patent Application No. 2012-154632 filed on July 10, 2012.

### [Technical Field]

The present invention relates to a water-in-oil emulsion cosmetic, and in particular, relates to a water-in-oil emulsion cosmetic that is excellent in the resilient and supple (in other words, firm, tensional, and elastic) feeling.

### [Background Art]

Emulsion compositions are broadly classified into an oil-in-water type (O/W) and a water-in-oil type (W/O). In addition, there are multiple types such as an oil-in-water-in-oil type (O/W/O) and a water-in-oil-in-water type (W/O/W). These have been utilized, in the past, for skin care cream, milky lotion, hair care cream, etc. in the cosmetic field.

Among them, the water-in-oil emulsion cosmetic, wherein the oil phase is the external phase and the water phase is the internal phase, is a suitable form as cosmetics because oil-soluble active components, for example, emollient oil, oil-soluble medicinal agents, UV absorbers, etc. can be effectively spread on the skin. In this respect, this is better than the oil-in-water type.

In recent years, it is desired that such a water-in-oil emulsion cosmetic provides a resilient and supple feeling (feeling in use wherein the skin does not sag, is not taut, and has moderate elasticity) to the skin.

On the other hand, for example, a monoester or diester of N-long chain acyl acidic amino acid is used in Patent Literature 1, and a dialkyl ester of N-acylated (10 to 30 carbons) glutamic acid is used in Patent Literature 2 to improve the feeling in use of water-in-oil emulsion compositions.

However, it has been especially difficult to bring out a resilient and supple feeling with the above components because the feeling in use is impaired depending upon the kinds and the blending quantities of moisturizing agents and oil, which are blended to achieve a moisturizing effect and other usability effect.

Patent Literature 3 discloses a water-in-oil type emulsified cosmetic having excellent moisture retention and preservation stability. Example 3 teaches a water-in-oil emulsion cosmetic comprising, among others, 4.0 mass% of di(phytosteryl/isostearyl/cetyl/stearyl/ behenyl) dimer dilinoleate, 25 mass% of methyltris(trimethylsiloxy)silane, 1.0 mass% of squalane, 1.0 mass% of diglyceryl monoisostearate, and 42.8 mass% of water.

Patent Literature 4 discloses a water-in-oil type cosmetic having excellent stability over time and being excellent in use feelings such as a wet body feeling, smooth extension, emollient feeling and non-skin-burdening feeling. Example 10 teaches a water-in-oil emulsion cosmetic comprising, among others, 3.0 mass% of (cholesteryl/behenyl/octyldodecyl) lauroyl glutamate, 10 mass% of octamethylcyclotetrasiloxane, 15 mass% of decamethylcylopentasiloxane, 2.0 mass% of squalane, 0.5 mass% of diglyceryl diisostearate, and 50.9 mass% of water.

Patent Literature 5 discloses a water-in-oil type emulsified cosmetic having excellent feeling of use and usability. Example 4 teaches a water-in-oil emulsion cosmetic comprising, among others, 0.5 mass% of cholesterol 12-hydroxystearate, 10 mass% of decamethylcylopentasiloxane, 5.0 mass% of glyceryl trioctanoate, 5.0 mass% of neopentyl glycol diisooctanoate, 0.5 mass% of polyoxyethylene-modified organopolysiloxane, and 50.1 mass% of water.

Patent Literature 6 discloses a water-in-oil type cosmetic composition providing a smooth feeling to the skin. Examples 1 to 5 teach water-in-oil emulsion cosmetics comprising, among others, 1.0 mass% of (cholesteryl/behenyl/octyldodecyl) lauroyl glutamate, 10 mass% of decamethylcylopentasiloxane, 10 mass% of squalane, 2.0 mass% of cetyl PEG/PPG-10/1 dimethicone, and about 47.5 to 52.5 mass% of water.

### [Prior Art Documents]

### [Patent Literatures]

[Patent Literature 1] Japanese Unexamined Patent Publication No. JP 2008-013515 A
[Patent Literature 2] Japanese Unexamined Patent Publication No. JP H05-279238 A
[Patent Literature 3] Japanese Unexamined Patent Publication No. JP 2005-104854 A
[Patent Literature 4] Japanese Unexamined Patent Publication No. JP 2002-255738 A
[Patent Literature 5] Japanese Unexamined Patent Publication No. JP H05-070335 A
[Patent Literature 6] International Patent Application Publication No. WO 2011/065772 A2

### [Summary of the Invention]

### [Problem to be Solved by the Invention]

The present invention was made in view of the above-described problems of the conventional art, and an object is to provide a water-in-oil emulsion cosmetic excellent in the resilient and supple feeling. The invention is defined by the claims.

### [Means to Solve the Problem]

The present inventors have diligently studied to solve the above-described problems; as a result, the present inventors have found that both the resilient and supple feeling and the moisturizing effect can be achieved by blending a sterol derivative and a volatile oil having low compatibility therewith to prepare a water-in-oil emulsion cosmetic; thus leading to the completion of the present invention.

That is, the water-in-oil emulsion cosmetic of the present invention comprises the following (A) to (D):
(A) 0.5 to 10 mass% of a sterol derivative selected from di(phytosteryl/octyldodecyl) lauroyl glutamate, macadamia nut oil fatty acid phytosteryl, di(octyldodecyl/phytosteryl/behenyl) lauroyl glutamate, myristoyl methyl-β-alanine (phytosteryl/decyltetradecyl), (phytosteryl/behenyl) dimer dilinoleate, (phytosteryl/isostearyl/cetyl/stearyl/behenyl) dimer dilinoleate, bis(behenyl/isostearyl/phytosteryl) dimer dilinoleyl dimer dilinoleate, di(isostearyl/phytosteryl) dimer dilinoleate, di(cholesteryl/behenyl/octyldodecyl) lauroyl glutamate, di(cholesteryl/octyldodecyl) lauroyl glutamate, and macadamia nut oil fatty acid cholesteryl,
(B) an oil containing (b1) a volatile oil having low compatibility with (A), and (b2) an oil with a viscosity of less than 1000 mPa•s other than (b1), wherein the volatile oil having low compatibility with (A) is selected from hexamethylcyclotrisiloxane, octamethylcyclotetrasiloxane, decamethylcyclopentasiloxane, dodecamethylcyclohexasiloxane, hexamethyldisiloxane, octamethyltrisiloxane, decamethyltetrasiloxane, dodecamethylpentasiloxane, tetradecamethylhexasiloxane, hexadecamethylheptasiloxane, methyltris(trimethylsiloxy)silane, tetrakis(trimethylsiloxy)silane, perfluoromethylcyclopentane, perfluorodimethylcyclohexane, methyl perfluorobutyl ether, methyl perfluoroisobutyl ether, ethyl perfluorobutyl ether, and ethyl perfluoroisobutyl ether,
(C) an emulsifying agent, and
(D) 65 to 85 mass% of an aqueous component which is normally usable in cosmetics, such as, in addition to water, moisturizing agents, water-soluble polymers, UV absorbers, sequestering agents, antioxidants and medicinal agents,
wherein the percentage of component (b1) with respect to component (A) and component (B) being 40 to 85 mass%, and wherein the blending quantity of component (b2) is two times or less of the blending quantity of component (A).

### [Effect of the Invention]

The water-in-oil emulsion cosmetic of the present invention is a cosmetic comprising a sterol derivative, a volatile oil having low compatibility with the aforementioned ester, an oil with a viscosity of less than 1000 mPa•s other than the volatile oil, an emulsifying agent, and an aqueous component and can provide a water-in-oil emulsion cosmetic excellent in the resilient and supple feeling.

### [Brief Description of the Drawings]

Fig. 1 shows the behavior of a sterol derivative and an additional oil (volatile oil that has high compatibility with the sterol derivative) on the skin.
Fig. 2 show the behavior of a sterol derivative and an additional oil (volatile oil that has low compatibility with the sterol derivative) on the skin.
Fig. 3 shows the behavior of a sterol derivative and an additional oil (nonvolatile oil that has low compatibility with the sterol derivative) on the skin.

### [Modes for Carrying Out the Invention]

The water-in-oil emulsion cosmetic of the present invention comprises (A) a sterol derivative, (B) an oil containing (b1) a volatile oil having low compatibility with the sterol derivative, (C) an emulsifying agent, and (D) an aqueous component.

Hereinafter, each component will be described in detail.

### (A) Sterol derivative

The sterol derivative, which is component (A) of the water-in-oil emulsion cosmetic of the present invention, is a sterol oil having a sterol skeleton. The sterol derivative is a phytosterol derivative and/or a cholesterol derivative, and may be used either alone or in combination of two or more.

Examples of the above-described phytosterol derivative include phytosterol-based oils such as acyl amino acid phytosterol/higher alcohol esters, dimer dilinoleate phytosterol/higher alcohol esters, and phytosterol fatty acid esters.

Examples of acyl amino acid phytosterol/higher alcohol esters include di(phytosteryl/octyldodecyl) lauroyl glutamate, di(octyldodecyl/phytosteryl/behenyl) lauroyl glutamate, and myristoyl methyl-β-alanine (phytosteryl/decyltetradecyl).

Examples of dimer dilinoleate phytosterol/higher alcohol esters include (phytosteryl/behenyl) dimer dilinoleate, (phytosteryl/isostearyl/cetyl/stearyl/behenyl) dimer dilinoleate, bis(behenyl/isostearyl/phytosteryl) dimer dilinoleyl dimer dilinoleate, and di(isostearyl/phytosteryl) dimer dilinoleate.

Examples of phytosterol fatty acid esters include macadamia nut oil fatty acid phytosteryl, lanolin fatty acid phytosteryl, phytosteryl stearate, phytosteryl isostearate, phytosteryl hydroxystearate, phytosteryl oleate, dihydrophytosteryl oleate, phytosteryl ricinolate, phytosteryl nonanoate, rice bran oil fatty acid phytosteryl, phytosteryl palmitate, phytosteryl 2-ethylhexanoate, phytosteryl caprate, phytosteryl laurate, and phytosteryl butyrate.

Examples of the commercial products of the above-described phytosterol derivatives include Eldew PS-203, Eldew PS-304, Eldew PS-306, Eldew APS-307 (all of them are manufactured by Ajinomoto Co., Inc.), YOFCO-MAS, Plandool-PB, Plandool-S, Plandool-G, and Lusplan PI-DA (all of them are manufactured by Nippon Fine Chemical Co., Ltd.).

Examples of the above-described cholesterol derivative include cholesterol-based oils selected from acyl amino acid cholesterol/higher alcohol esters and cholesterol fatty acid esters.

Examples of acyl amino acid cholesterol/higher alcohol esters include di(cholesteryl/behenyl/octyldodecyl) lauroyl glutamate and di(cholesteryl/octyldodecyl) lauroyl glutamate.

Examples of cholesterol fatty acid esters include macadamia nut oil fatty acid cholesteryl, cholesteryl hydroxystearate, lanolin fatty acid cholesteryl, branched fatty acid (C12-31) cholesteryl, cholesteryl stearate, cholesteryl isostearate, cholesteryl oleate, dihydrocholesteryl oleate, cholesteryl ricinolate, and cholesteryl nonanoate.

Examples of the commercial products of the above-described cholesterol derivatives include Eldew CL-301, Eldew CL-202 (all of them are manufactured by Ajinomoto Co., Inc.), Estemol CHS (manufactured by Nisshin OilliO Group, Ltd.), YOFCO-MAC, YOFCO CLE-S, and YOFCO CLE-NH (all of them are manufactured by Nippon Fine Chemical Co., Ltd.).

According to the invention, however, one or more selected from di(phytosteryl/octyldodecyl) lauroyl glutamate, macadamia nut oil fatty acid phytosteryl, di(octyldodecyl/phytosteryl/behenyl) lauroyl glutamate, myristoyl methyl-β-alanine (phytosteryl/decyltetradecyl), (phytosteryl/behenyl) dimer dilinoleate, (phytosteryl/isostearyl/cetyl/stearyl/behenyl) dimer dilinoleate, bis(behenyl/isostearyl/phytosteryl) dimer dilinoleyl dimer dilinoleate, di(isostearyl/phytosteryl) dimer dilinoleate, di(cholesteryl/behenyl/octyldodecyl) lauroyl glutamate, di(cholesteryl/octyldodecyl) lauroyl glutamate, and macadamia nut oil fatty acid cholesteryl are blended as the above-described sterol derivative.

It is necessary that the blending quantity of the (A) sterol derivative in the water-in-oil emulsion cosmetic of the present invention is 0.5 to 10 mass% with respect to the total amount of the cosmetic. Furthermore, it is preferable that the blending quantity is 1 to 5 mass%. If the blending quantity of component (A) is less than 0.5 mass%, the resilient and supple feeling and the moisturizing effect cannot satisfactorily be obtained. If the blending quantity of component (A) exceeds 10 mass%, the usability such as non-stickiness and softness is poor and the stability is poor.

### (B) Oil

It is necessary that the (B) oil as used in the present invention comprises component (b1), namely a volatile oil that has low compatibility with the (A) sterol derivative.

Here, the oil of low compatibility with the sterol derivative means an oil that does not become a homogeneous transparent layer when the sterol derivative and the target oil are mixed, heated to 80 °C, and brought back to room temperature. The volatile oil is an oil whose boiling point is 300 °C or lower at 1 atm.

According to the invention, component (b1) is selected from hexamethylcyclotrisiloxane, octamethylcyclotetrasiloxane, decamethylcyclopentasiloxane, dodecamethylcyclohexasiloxane, hexamethyldisiloxane, octamethyltrisiloxane, decamethyltetrasiloxane, dodecamethylpentasiloxane, tetradecamethylhexasiloxane, hexadecamethylheptasiloxane, methyltris(trimethylsiloxy)silane, tetrakis(trimethylsiloxy)silane, perfluoromethylcyclopentane, perfluorodimethylcyclohexane, methyl perfluorobutyl ether, methyl perfluoroisobutyl ether, ethyl perfluorobutyl ether, and ethyl perfluoroisobutyl ether.

Furthermore, the blending quantity of the (b1) volatile oil having low compatibility with the sterol derivative in the water-in-oil emulsion cosmetic of the present invention is preferably 6 to 20 mass% with respect to the total amount of the cosmetic, more preferably 10 to 20 mass%, and especially preferably 13 to 18 mass%. If the blending quantity of component (b1) is too small, the resilient and supple feeling may be poor. If the blending quantity of component (b1) is too large, usability may be poor.

In the water-in-oil emulsion cosmetic of the present invention, (b2) an oil with a viscosity of less than 1000 mPa•s in addition to component (b1) is blended. In the present invention, the viscosity is a value measured at ordinary temperature (25 °C) with a viscometer (measurement conditions with a viscometer: BL-type, 12 rpm, and rotor No. 2).

Examples of the (b2) oil with a viscosity of less than 1000 mPa•s include silicone oil, polar oil, and nonpolar oil.

Examples of silicone oil include linear silicone oils such as methylpolysiloxane, methylphenylpolysiloxane, and methylhydrogenpolysiloxane; and cyclic silicone oils.

Examples of polar oils include ester oils, other than component (A), such as cetyl octanoate (cetyl ethylhexanoate), hexyl laurate, isopropyl myristate, octyl palmitate, isocetyl stearate, isopropyl isostearate, octyl isopalmitate, isodecyl isostearate, 2-ethylhexyl succinate, and diethyl sebacate.

Examples of nonpolar oils include hydrocarbon oils such as liquid paraffin, squalane, squalene, paraffin, and isohexadecane.

In the water-in-oil emulsion cosmetic of the present invention, the blending quantity of component (b2) is two times or less of the blending quantity of component (A). If the blending quantity of component (b2) exceeds two times of the blending quantity of component (A), a satisfactory resilient and supple feeling may not be obtained.

In the cosmetic of the present invention, it is also preferable to further blend (b3) a high-viscosity oil.

In the present invention, the (b3) high-viscosity oil means an oil selected from solid oil, semi-solid oil other than component (A), and the oil with a viscosity of 1000 mPa•s or higher. These oils may be blended alone or two or more may be blended.

By blending the high-viscosity oil, a water-in-oil emulsion cosmetic excellent in the resilient and supple feeling and the moisturizing effect can be obtained. The blending quantity of the high-viscosity oil should be in the amount that the stability of the emulsion cosmetic is not affected.

Examples of the solid oil, among (b3) high-viscosity oils, include solid fats such as cocoa butter, coconut oil, horse oil, hydrogenated coconut oil, palm oil, beef tallow, mutton tallow, and hydrogenated castor oil; hydrocarbons such as paraffin wax (linear hydrocarbon), microcrystalline wax (branched saturated hydrocarbon), ceresin wax, japan wax, montan wax, and Fischer-Tropsch wax; waxes such as beeswax, carnauba wax, candelilla wax, rice bran wax (rice wax), spermaceti wax, jojoba oil, rice bran wax, montan wax, kapok wax, bayberry wax, shellac wax, sugar cane wax, lanolin fatty acid isopropyl, hexyl laurate, reduced lanolin, hard lanolin, POE lanolin alcohol ether, POE lanolin alcohol acetate, POE cholesterol ether, lanolin fatty acid polyethylene glycol, and POE hydrogenated lanolin alcohol ether; higher fatty acids such as myristic acid, palmitic acid, stearic acid, and behenic acid; and higher alcohols such as cetyl alcohol, stearyl alcohol, behenyl alcohol, myristyl alcohol, and cetostearyl alcohol.

Example of semi-solid oil other than component (A) include petrolatum, lanolin, shea butter, vegetable tallow such as partially hydrogenated coconut oil, partially hydrogenated jojoba oil, in addition, pentaerythrityl tetra(behenate/benzoate/ethylhexanoate), macadamia nut oil polyglyceryl-6 esters behenate, and dipentaerythrityl hexaoxystearate.

Examples of oil with a viscosity of 1000 mPa•s or higher include glyceryl isostearate, diisostearyl malate, hydrogenated lanolin, and hydrogenated polyisobutene.

In the external phase (oil phase) of the water-in-oil emulsion cosmetic of the present invention, it is preferable that the (A) sterol derivative is dispersed in the continuous-phase component (b1).

Component (A) has low compatibility with component (b1); however, in the emulsion cosmetic of the present invention, in which the blending quantity of the internal water phase is large, component (A) can be stably micro-dispersed in component (b1) without separation.

In the water-in-oil emulsion cosmetic of the present invention, it is necessary that the percentage of component (b1) with respect to component (A) and component (B) (amount of (bl)/amount of (A) + (B)) is 40 to 85%. Furthermore, it is preferable that the percentage is 55 to 85%. If the percentage of component (b1) with respect to component (A) and component (B) is less than 40%, a satisfactory resilient and supple feeling may not be obtained. If the percentage of component (b1) with respect to component (A) and component (B) exceeds 85%, the stability and usability may be poor.

### (C) Emulsifying agent

As the (C) emulsifying agent, those normally usable in cosmetics can be used.

It is especially preferable to use an emulsifying agent whose HLB is 5 or lower. An emulsifying agent whose HLB exceeds 5 has high hydrophilicity, and it may be difficult to obtain a stable water-in-oil emulsion cosmetic.

The above HLB value can be calculated according to the Kawakami equation represented by HLB = 7 + 11.7 • log(MW/MO) (here, MW represents the molecular weight of the hydrophilic group part and MO represents the molecular weight of the lipophilic group part).

Examples of the emulsifying agent include organically modified clay minerals, silicone surfactants, and polyhydric alcohol fatty acid ester surfactants.

Examples of organically modified clay minerals include dimethylalkylammonium hectorite, benzyldimethylstearylammonium hectorite, and distearyldimethylammonium chloride-treated aluminum magnesium silicate.

Examples of silicone surfactants include poly(oxyethylene/oxypropylene) methylpolysiloxane copolymer, polyoxyethylene methylpolysiloxane copolymer, silicone-chain branched-type methylpolysiloxane copolymer, alkyl-chain branched-type polyoxyethylene methylpolysiloxane copolymer, alkyl-chain/silicone-chain branched-type polyoxyethylene methylpolysiloxane copolymer, crosslinked-type polyoxyethylene methylpolysiloxane, alkyl group-containing crosslinked-type polyoxyethylene methylpolysiloxane, branched-type polyglycerin-modified silicone, and alkyl-group branched-type polyglycerin-modified silicone.

Examples of polyhydric alcohol fatty acid ester surfactants include glycerin fatty acid esters, polyglycerin fatty acid esters, polyoxyethylene glycerin fatty acid esters, sorbitan fatty acid esters, and polyoxyethylene sorbitan fatty acid esters.

It is preferable that the blending quantity of the (C) emulsifying agent in the water-in-oil emulsion cosmetic of the present invention is 0.5 to 5 mass% with respect to the total amount of the cosmetic. Furthermore, it is especially preferable that the blending quantity is 1 to 4 mass%. If the blending quantity of component (C) is too small, the stability may be poor. If the blending quantity of component (C) is too large, the usability may be poor.

### (D) Aqueous component

The (D) aqueous component, which is normally usable in cosmetics, can be blended within the range that the stability of the emulsion is not impaired.

Examples of the (D) aqueous component include, in addition to water, moisturizing agents, water-soluble polymers, UV absorbers, sequestering agents, antioxidants, and medicinal agents.

Examples of moisturizing agents include 1,3-butylene glycol, polyethylene glycol, propylene glycol, dipropylene glycol, hexylene glycol, glycerin, diglycerin, xylitol, maltitol, maltose, and D-mannitol. Examples of water-soluble polymers include plant-based polymers such as gum arabic, carrageenan, pectin, agar, quince seed (Cydonia oblonga), starch, and algae colloid (brown algae extract); microorganism-based polymers such as dextran and pullulan; animal-based polymers such as collagen, casein, and gelatin; starch-based polymers such as carboxymethyl starch and methylhydroxypropyl starch; alginic acid-based polymers such as sodium alginate; vinyl-based polymers such as carboxyvinyl polymer (CARBOPOL etc.); polyoxyethylene-based polymers; polyoxyethylene/polyoxypropylene copolymer-based polymers; acryl-based polymers such as sodium polyacrylate and polyacrylamide; and inorganic water-soluble polymers such as bentonite, aluminum magnesium silicate, and laponite.

Examples of sequestering agents include sodium edetate, sodium metaphophate, and phosphoric acid. An example of antioxidant includes ascorbic acid.

Examples of medicinal agents include vitamins such as vitamin D2 (ergocalciferol), and dl-α-tocopheryl acetate; astringents such as zinc oxide and sulfur. The above-described medicinal agents are used in a free state; in addition, the salt-formable agents can be used in a salt form with acid or base, and the agents having a carboxylic group can be used as an ester form.

It is necessary that the blending quantity of the (D) aqueous component in the water-in-oil emulsion cosmetic of the present invention is 65 to 85 mass% with respect to the total amount of the cosmetic. If the blending quantity of component (D) is too small, the separation or precipitation of the sterol derivative takes place and the usability of the cosmetic is poor. If the blending quantity of component (D) is too large, the stability is poor.

The water-in-oil emulsion cosmetic of the present invention can be widely utilized in the cosmetics that are usually applied to the outer skin. Examples include products such as beauty whitening essence, milky lotion, cream, pack, foundation, lipstick, eyeshadow, eyeliner, mascara, facial cleanser, spray, mousse, hair rinse, and shampoo.

### [Examples]

The present invention will hereinafter be explained in further detail with reference to examples; however, the present invention is by no means limited by these examples. The blending quantity is expressed, unless otherwise noted, by mass% with respect to the system wherein the component is blended.

Prior to the description of examples, test evaluation methods used in this application are explained.

### Evaluation (1): Dispersion stability

The sample appearance was visually evaluated one week after preparation.
A: The separation of oil was not observed.
B: Oil separated and precipitation was observed.
C: Oil separated within a week and precipitation was observed.

### Evaluation (2): Resilient and supple feeling

Ten professional panelists applied a sample on the face and evaluated the feeling in use upon application.
A*: Among 10 panelists, 9 or more panelists answered that a resilient and supple feeling was present.
A: Among 10 panelists, 7 or more and less than 9 panelists answered that a resilient and supple feeling was present.
B: Among 10 panelists, 5 or more and less than 7 panelists answered that a resilient and supple feeling was present.
C: Among 10 panelists, less than 5 panelists answered that a resilient and supple feeling was present.

### Evaluation (3): Stability

The stability was evaluated by comparing the hardness and appearance of a sample stored for 1 month at 25 °C and 40 °C with those immediately after preparation.
A*: Under all the storage conditions, the decrease in hardness was 10% or lower, and the change in appearance was not observed.
A: Under all the storage conditions, the change in appearance was not observed; however, 10% or higher decrease in hardness was observed only for the sample stored at 40 °C.
B*: Under all the storage conditions, the change in appearance was not observed; however, 10% or higher decrease in hardness was observed.
B: In appearance, the separation of water or oil was somewhat observed.
C: Within 1 month, the separation of water or oil was observed in appearance.

### Evaluation (4): Plump feeling

Ten professional panelists applied a sample on the face and evaluated the feeling in use upon application.
A*: Among 10 panelists, 9 or more panelists answered that a plump feeling was present.
A: Among 10 panelists, 7 or more and less than 9 panelists answered that a plump feeling was present.
B: Among 10 panelists, 5 or more and less than 7 panelists answered that a plump feeling was present.
C: Among 10 panelists, less than 5 panelists answered that a plump feeling was present.

### Evaluation (5): Softness

Ten professional panelists applied a sample on the face and evaluated the feeling in use upon application.
A*: Among 10 panelists, 9 or more panelists answered that it was soft.
A: Among 10 panelists, 7 or more and less than 9 panelists answered that it was soft.
B: Among 10 panelists, 5 or more and less than 7 panelists answered that it was soft.
C: Among 10 panelists, less than 5 panelists answered that it was soft.

### Evaluation (6): Moisturizing effect

Ten professional panelists applied a sample on the face and evaluated the feeling in use upon application.
A*: Among 10 panelists, 9 or more panelists answered that a moisturizing effect was present.
A: Among 10 panelists, 7 or more and less than 9 panelists answered that a moisturizing effect was present.
B: Among 10 panelists, 5 or more and less than 7 panelists answered that a moisturizing effect was present.
C: Among 10 panelists, less than 5 panelists answered that a moisturizing effect was present.

The present inventors have investigated the resilient and supple feeling by using the sterol derivatives di(phytosteryl/2-octyldodecyl) N-lauroyl-L-glutamate and macadamia nut oil fatty acid phytosteryl as the polymer having high adhesion to the skin with a resilient and supple feeling. Di(phytosteryl/2-octyldodecyl) N-lauroyl-L-glutamate is a high-viscosity liquid (viscosity 1,640 mPa•s) and macadamia nut oil fatty acid phytosteryl is a semi-solid at ordinary temperature; therefore, it is difficult to blend them alone in the cosmetic.

Therefore, the cosmetics shown in the below Tables 1 and 2, wherein di(phytosteryl/2-octyldodecyl) N-lauroyl-L-glutamate or macadamia nut oil fatty acid phytosteryl are blended with various additional oils, were produced. Then, each sample was evaluated, for evaluation items (1) and (2), according to the above-described scoring criteria. The results are shown in Tables 1 and 2.

In the following tests, "compatibility (*1)" represents the compatibility of the sterol derivative and the oil. That is, when two kinds of oils, namely the sterol derivative and the additional oil, are mixed, heated to 80 °C, and brought back to room temperature, it is evaluated to be "○" in the case that a homogeneous transparent layer is formed and "x" in the case that a homogeneous transparent layer is not formed.

**[Table 1]**

| Test Example | | | 1-1* | 1-2* | 1-3* | 1-4* | 1-5* |
|---|---|---|---|---|---|---|---|
| | Compatibility (*1) | Property | | | | | |
| Di(phytosteryl/2-octyldodecyl)N-lauroyl-L-glutamate | | | 50 | 50 | 50 | 50 | 50 |
| Isoparaffin | ○ | volatile | 50 | - | - | - | - |
| Squalane | ○ | nonvolatile | - | 50 | - | - | - |
| Decamethylcyclopentasiloxane | × | volatile | - | - | 50 | - | - |
| Decamethyltetrasiloxane | × | volatile | - | - | - | 50 | - |
| Dimethylpolysiloxane 6cs | × | nonvolatile | - | - | - | - | 50 |
| Evaluation (1): Dispersion stability | | | A | A | C | C | C |
| Evaluation (2): Resilient and supple feeling | | | C | C | A | A | C |

| | | | | | | | |
|---|---|---|---|---|---|---|---|
| * (not according to the invention) | | | | | | | |

**[Table 2]**

| Test Example | | | 1-6* | 1-7* | 1-8* | 1-9* | 1-10* |
|---|---|---|---|---|---|---|---|
| | Compatibility (*1) | Property | | | | | |
| Macadamia nut oil fatty acid phytosteryl | | | 50 | 50 | 50 | 50 | 50 |
| Isoparaffin | ○ | volatile | 50 | - | - | - | - |
| Squalane | ○ | nonvolatile | - | 50 | - | - | - |
| Decamethylcyclopentasiloxane | × | volatile | - | - | 50 | - | - |
| Decamethyltetrasiloxane | × | volatile | - | - | - | 50 | - |
| Dimethylpolysiloxane 6cs | × | nonvolatile | - | - | - | - | 50 |
| Evaluation (1): Dispersion stability | | | A | A | C | C | C |
| Evaluation (2): Resilient and supple feeling | | | C | C | A | A | C |

| | | | | | | | |
|---|---|---|---|---|---|---|---|
| * (not according to the invention) | | | | | | | |

Test Examples 1-1, 1-2, 1-6, and 1-7, which are oil-based cosmetics, wherein oil (isoparaffin or squalane) that can normally dissolve di(phytosteryl/2-octyldodecyl) N-lauroyl-L-glutamate or macadamia nut oil fatty acid phytosteryl was blended, were stable; however, no resilient and supple feeling was present.

In Test Examples 1-3 to 1-5 and 1-8 to 1-10, wherein di(phytosteryl/2-octyldodecyl) N-lauroyl-L-glutamate or macadamia nut oil fatty acid phytosteryl and an oil of low compatibility therewith (decamethylcyclopentasiloxane, decamethyltetrasiloxane, or dimethylpolysiloxane) were blended, di(phytosteryl/2-octyldodecyl) N-lauroyl-L-glutamate or macadamia nut oil fatty acid phytosteryl precipitated over time, and the stability was poor. However, the samples of Test Examples 1-3, 1-4, 1-8, and 1-9, wherein a volatile oil was blended, were excellent in the resilient and supple feeling.

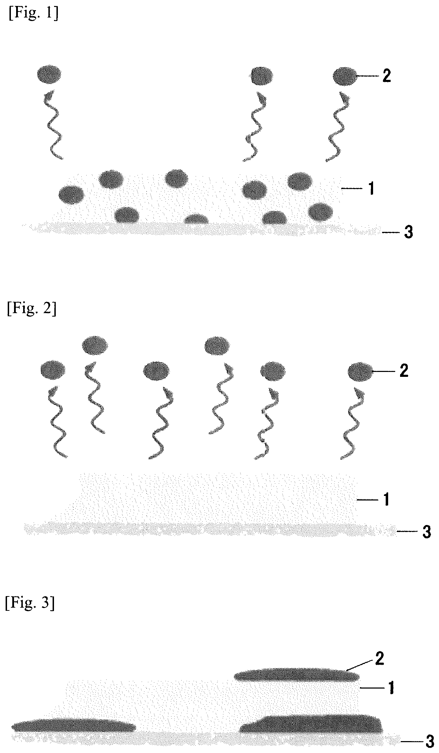

The present inventors investigated the behavior on the skin of the oil (sterol derivative: di(phytosteryl/2-octyldodecyl) N-lauroyl-L-glutamate and macadamia nut oil fatty acid phytosteryl), which achieves a resilient and supple feeling, and an additional oil that is blended therewith in the cosmetic. The results are shown in Fig. 1 to Fig. 3.

As shown in Test Examples 1-1 and 1-6, when a volatile oil (isoparaffin) having high compatibility with di(phytosteryl/2-octyldodecyl) N-lauroyl-L-glutamate and macadamia nut oil fatty acid phytosteryl is used as the additional oil, the behavior shown in Fig. 1 is considered to be displayed. That is, because isoparaffin has high compatibility with di(phytosteryl/2-octyldodecyl) N-lauroyl-L-glutamate and macadamia nut oil fatty acid phytosteryl, part of isoparaffin evaporates but part of isoparaffin coexists, and a low concentration of di(phytosteryl/2-octyldodecyl) N-lauroyl-L-glutamate or macadamia nut oil fatty acid phytosteryl is considered to be coated on the skin.

As shown in Test Examples 1-3 and 1-8, when a volatile oil (decamethylcyclopentasiloxane) having low compatibility with di(phytosteryl/2-octyldodecyl) N-lauroyl-L-glutamate and macadamia nut oil fatty acid phytosteryl is used as the additional oil, the behavior shown in Fig. 2 is considered to be displayed. That is, because decamethylcyclopentasiloxane has low compatibility with di(phytosteryl/2-octyldodecyl) N-lauroyl-L-glutamate and macadamia nut oil fatty acid phytosteryl, the volatile oil decamethylcyclopentasiloxane immediately separates and easily evaporates when applied on the skin. Accordingly, a large amount of di(phytosteryl/2-octyldodecyl) N-lauroyl-L-glutamate or macadamia nut oil fatty acid phytosteryl is considered to adhere on the skin.

As shown in Test Examples 1-5 and 1-10, when a nonvolatile oil (dimethylpolysiloxane) having low compatibility with di(phytosteryl/2-octyldodecyl) N-lauroyl-L-glutamate and macadamia nut oil fatty acid phytosteryl is used as the additional oil, the behavior shown in Fig. 3 is considered to be displayed. That is, because dimethylpolysiloxane has low compatibility with di(phytosteryl/2-octyldodecyl) N-lauroyl-L-glutamate and macadamia nut oil fatty acid phytosteryl and it is nonvolatile, di(phytosteryl/2-octyldodecyl) N-lauroyl-L-glutamate or macadamia nut oil fatty acid phytosteryl and dimethylpolysiloxane are non-uniform when applied on the skin ; thus it is considered that each of them aggregates and adheres to the skin and unevenness is generated.

From Tables 1 and 2 and Figs. 1 to 3, it is considered that the adhesion to the skin and the frictional property, which di(phytosteryl/2-octyldodecyl) N-lauroyl-L-glutamate and macadamia nut oil fatty acid phytosteryl have, are achieved in the system wherein a large amount of di(phytosteryl/2-octyldodecyl) N-lauroyl-L-glutamate or macadamia nut oil fatty acid phytosteryl adheres when applied on the skin, and a cosmetic excellent in the resilient and supple feeling is obtained.

That is, as shown in 1-3, 1-4, 1-8, and 1-9, it was clarified that the finning effect of a sterol derivative can be maximized by concurrently using the sterol derivative such as di(phytosteryl/2-octyldodecyl) N-lauroyl-L-glutamate or macadamia nut oil fatty acid phytosteryl and a volatile oil having low compatibility therewith.

However, when a sterol derivative and a volatile oil having low compatibility therewith are mixed, a cosmetic that is satisfactory in stability cannot be obtained as described above.

Thus, the present inventors blended these oils in the water-in-oil emulsion cosmetic and tried to suppress the separation of the sterol derivative in the oil phase.

That is, the present inventors produced water-in-oil emulsion cosmetics (cream) with the blending compositions shown in the below Tables 3 and 4, wherein di(phytosteryl/2-octyldodecyl) N-lauroyl-L-glutamate or macadamia nut oil fatty acid phytosteryl is blended and the kinds of concurrently used additional oils are varied, by the conventional method. Then, each sample was evaluated, for evaluation items (2) to (6), according to the above-described scoring criteria. The results are shown in Tables 3 and 4.

**[Table 3]**

| Test Example | | | 2-1* | 2-2* | 2-3* | 2-4* |
|---|---|---|---|---|---|---|
| | compatibility | Property | | | | |
| Di(phytosteryl/2-octyldodecyl) N-lauroyl-L-glutamate | | | 5 | 5 | 5 | 5 |
| Decamethyltetrasiloxane | × | volatile | 20 | - | - | - |
| Isohexadecane | ○ | volatile | - | 20 | - | - |
| Methylpolysiloxane | × | nonvolatile | - | - | 20 | - |
| Squalane | ○ | nonvolatile | - | - | - | 20 |
| Dimethyl distearyl ammonium modified hectorite | | | 1.7 | 1.7 | 1.7 | 1.7 |
| Polyoxyethylene/methylpolysiloxane copolymer | | | 0.5 | 0.5 | 0.5 | 0.5 |
| Ion-exchanged water | | | 64.3 | 64.3 | 64.3 | 64.3 |
| Glycerin | | | 8 | 8 | 8 | 8 |
| Sodium chloride | | | 0.5 | 0.5 | 0.5 | 0.5 |
| Evaluation (2): Resilient and supple feeling | | | A* | B | B | C |
| Evaluation (3): Stability | | | A | A | A | A |
| Evaluation (4): Plump feeling | | | A | B | B | B |
| Evaluation (5): Softness | | | A | B | B | B |
| Evaluation (6): Moisturizing effect | | | B | A | B | A |

| | | | | | | |
|---|---|---|---|---|---|---|
| * (not according to the invention) | | | | | | |

**[Table 4]**

| Test Example | | | 2-5* | 2-6* | 2-7* | 2-8* |
|---|---|---|---|---|---|---|
| | compatibility | Property | | | | |
| Macadamia nut oil fatty acid phytosteryl | | | 5 | 5 | 5 | 5 |
| Decamethyltetrasiloxane | × | volatile | 20 | - | - | - |
| Isohexadecane | ○ | volatile | - | 20 | - | - |
| Methylpolysiloxane | × | nonvolatile | - | - | 20 | - |
| Squalane | ○ | nonvolatile | - | - | - | 20 |
| Dimethyl distearyl ammonium modified hectorite | | | 1.7 | 1.7 | 1.7 | 1.7 |
| Polyoxyethylene/methylpolysiloxane copolymer | | | 0.5 | 0.5 | 0.5 | 0.5 |
| Ion-exchanged water | | | 64.3 | 64.3 | 64.3 | 64.3 |
| Glycerin | | | 8 | 8 | 8 | 8 |
| Sodium chloride | | | 0.5 | 0.5 | 0.5 | 0.5 |
| Evaluation (2): Resilient and supple feeling | | | A* | B | B | C |
| Evaluation (3): Stability | | | A | A | A | A |
| Evaluation (4): Plump feeling | | | A | B | B | B |
| Evaluation (5): Softness | | | A | B | B | B |
| Evaluation (6): Moisturizing effect | | | B | A | B | A |

| | | | | | | |
|---|---|---|---|---|---|---|
| * (not according to the invention) | | | | | | |

According to Tables 3 and 4, the resilient and supple feeling was excellent in Test Examples 2-1 and 2-5 wherein di(phytosteryl/2-octyldodecyl) N-lauroyl-L-glutamate or macadamia nut oil fatty acid phytosteryl and a volatile oil having low compatibility therewith are blended. Furthermore, it is seen that the stability is improved, by allowing them to be water-in-oil emulsion cosmetics, compared with Test Examples 1-4 and 1-9. Thus, it is suggested that di(phytosteryl/2-octyldodecyl) N-lauroyl-L-glutamate or macadamia nut oil fatty acid phytosteryl in the oil phase is stably dispersed in the additional continuous-phase oil without separation (precipitation) by allowing to be a water-in-oil type emulsion system.

On the other hand, the resilient and supple feeling was not satisfactory with water-in-oil emulsion cosmetics wherein in addition to di(phytosteryl/2-octyldodecyl) N-lauroyl-L-glutamate or macadamia nut oil fatty acid phytosteryl, a volatile oil having high compatibility therewith (Test Examples 2-2 and 2-6) or a nonvolatile oil having high or low compatibility therewith (Test Examples 2-3, 2-4, 2-7, and 2-8) are blended,.

Thus, it was clarified that a stable cosmetic can be obtained, without impairing the resilient and supple feeling that the sterol derivative exhibits, by allowing (A) a sterol derivative and (B) (b1) a volatile oil having low compatibility with the sterol derivative to be a water-in-oil emulsion cosmetic with (C) an emulsifying agent and (D) an aqueous component.

As a result of further investigation by the present inventors, when the blending quantity of decamethyltetrasiloxane is increased to 40 mass% in Test Examples 2-1 and 2-5, the separation of the sterol derivative was observed over time, and it was "C" in evaluation item (3). Accordingly, in the water-in-oil emulsion cosmetic of the present invention, it is necessary that the blending quantity of the (D) aqueous component is 60 mass% or higher.

Subsequently, other components effective for the further improvement of stability and usability were investigated. The present inventors produced, by the conventional method, water-in-oil emulsion cosmetics (cream) with the blending compositions shown in the below Tables 5 and 6. Then, each sample was evaluated, for evaluation items (2) to (6), according to the above-described scoring criteria. The results are shown in Tables 5 and 6.

**[Table 5]**

| Test Example | | | 3-1* | 3-2 | 2-1* | 3-3 | 3-4 | 3-5* | 3-6* |
|---|---|---|---|---|---|---|---|---|---|
| (A) | | Di(phytosteryl/2-octyldodecyl) N-lauroyl-L-glutamate | 2 | 2 | 5 | 5 | 5 | 5 | 5 |
| (B) | (b1) | Decamethyltetrasiloxane | 19.5 | 18 | 20 | 17 | 14 | 9 | 5 |
| | | Squalane | 0.5 | 2 | - | 3 | 6 | 11 | 15 |
| (C) | | Dimethyl distearyl ammonium modified hectorite | 1.7 | 1.7 | 1.7 | 1.7 | 1.7 | 1.7 | 1.7 |
| | | Polyoxyethylene/methylpolysiloxane copolymer | 0.5 | 0.5 | 0.5 | 0.5 | 0.5 | 0.5 | 0.5 |
| (D) | | Ion-exchanged water | 67.3 | 67.3 | 64.3 | 64.3 | 64.3 | 64.3 | 64.3 |
| | | Glycerin | 8 | 8 | 8 | 8 | 8 | 8 | 8 |
| | | Sodium chloride | 0.5 | 0.5 | 0.5 | 0.5 | 0.5 | 0.5 | 0.5 |
| Blending quantity of (A)+(B) | | | 22 | 22 | 25 | 25 | 25 | 25 | 25 |

| Blending quantity of (b1) / Blending quantity of (A)+(B) (%) | | | 89 | 82 | 80 | 68 | 56 | 36 | 20 |
|---|---|---|---|---|---|---|---|---|---|
| Evaluation (2): Resilient and supple feeling | | | A* | A* | A* | A* | A | B | C |
| Evaluation (3): Stability | | | A | A | A | A | A* | A* | A* |
| Evaluation (4): Plump feeling | | | B | A | A | A | A | A | B |
| Evaluation (5): Softness | | | B | A | A | A | A | A | B |
| Evaluation (6): Moisturizing effect | | | B | A | B | A | A | A | A |

| | | | | | | | | | |
|---|---|---|---|---|---|---|---|---|---|
| * (not according to the invention) | | | | | | | | | |

**[Table 6]**

| Test Example | | | 3-7* | 3-8 | 2-5* | 3-9 | 3-10 | 3-11* | 3-12* |
|---|---|---|---|---|---|---|---|---|---|
| (A) | | Macadamia nut oil fatty acid phytosteryl | 2 | 2 | 5 | 5 | 5 | 5 | 5 |
| (B) | (b1) | Decamethyltetrasiloxane | 19.5 | 18 | 20 | 17 | 14 | 9 | 5 |
| | | Squalane | 0.5 | 2 | - | 3 | 6 | 11 | 15 |
| (C) | | Dimethyl distearyl ammonium modified hectorite | 1.7 | 1.7 | 1.7 | 1.7 | 1.7 | 1.7 | 1.7 |
| | | Polyoxyethylene/methylpolysiloxane copolymer | 0.5 | 0.5 | 0.5 | 0.5 | 0.5 | 0.5 | 0.5 |
| (D) | | Ion-exchanged water | 67.3 | 67.3 | 64.3 | 64.3 | 64.3 | 64.3 | 64.3 |
| | | Glycerin | 8 | 8 | 8 | 8 | 8 | 8 | 8 |
| | | Sodium chloride | 0.5 | 0.5 | 0.5 | 0.5 | 0.5 | 0.5 | 0.5 |
| Blending quantity of (A)+(B) | | | 22 | 22 | 25 | 25 | 25 | 25 | 25 |
| Blending quantity of (b1) / Blending quantity of (A)+(B) | | | 89 | 82 | 80 | 68 | 56 | 36 | 20 |
| Evaluation (2): Resilient and supple feeling | | | A* | A* | A* | A* | A | B | C |
| Evaluation (3): Stability | | | A | A | A | A | A* | A* | A* |
| Evaluation (4): Plump feeling | | | B | A | A | A | A | A | B |
| Evaluation (5): Softness | | | B | A | A | A | A | A | B |
| Evaluation (6): Moisturizing effect | | | B | A | B | A | A | A | A |

| | | | | | | | | | |
|---|---|---|---|---|---|---|---|---|---|
| * (not according to the invention) | | | | | | | | | |

According to Test Examples 3-2 to 3-4 and 3-8 to 3-10, wherein squalane was suitably blended to the samples of Test Examples 2-1 and 2-5 wherein components (A) to (D) were suitably blended, it is seen that the stability and usability are improved by blending squalane.

As a result of further investigation by the present inventors, it was clarified that the oil represented by squalane, which is effective for the improvement of the stability and usability, is an oil with a viscosity of less than 1000 mPa•s.

In addition, it was found that the resilient and supple feeling and usability are affected by the percentage of the (b1) volatile oil having low compatibility with the sterol derivative with respect to component (A) and component (B).

Accordingly, the water-in-oil emulsion cosmetic of the present invention contains (b2) an oil with a viscosity of less than 1000 mPa•s.

Furthermore, it is necessary that the percentage of the (b1) volatile oil having low compatibility with the sterol derivative is 40 to 85% with respect to component (A) and component (B).

Subsequently, the blending quantities of various oils were investigated. The present inventors produced water-in-oil emulsion cosmetics (cream) by the conventional method according to the blending compositions, wherein the blending quantities of various oils were varied, shown in the below Tables 7 and 8. Then, each sample was evaluated, for evaluation items (2) to (6), according to the above-described scoring criteria. The results are shown in Tables 7 and 8.

**[Table 7]**

| Test Example | | | 3-4 | 4-1* | 4-2 | 4-3 |
|---|---|---|---|---|---|---|
| (A) | | Di(phytosteryl/2-octyldodecyl) N-lauroyl-L-glutamate | 5 | 2 | 2 | 5 |
| (B) | (b1) | Decamethyltetrasiloxane | 14 | 12 | 12 | 17 |
| | (b2) | Squalane | 6 | 5 | 4 | 6 |
| (C) | | Dimethyl distearyl ammonium modified hectorite | 1.7 | 1.7 | 1.7 | 1.7 |
| | | Polyoxyethylene/methylpolysiloxane copolymer | 0.5 | 0.5 | 0.5 | 0.5 |
| (D) | | Ion-exchanged water | 64.3 | 70.3 | 71.3 | 61.3 |
| | | Glycerin | 8 | 8 | 8 | 8 |
| | | Sodium chloride | 0.5 | 0.5 | 0.5 | 0.5 |
| Blending quantity of (A)+(B) | | | 25 | 19 | 18 | 28 |
| Blending quantity of (b1) / Blending quantity of (A)+(B) (%) | | | 56 | 63 | 67 | 61 |
| Evaluation (2): Resilient and supple feeling | | | A | B | A | A |
| Evaluation (3): Stability | | | A* | A* | A* | A* |
| Evaluation (4): Plump feeling | | | A | B | A | A |
| Evaluation (5): Softness | | | A | B | A | A |
| Evaluation (6): Moisturizing effect | | | A | A | A | A |

| | | | | | | |
|---|---|---|---|---|---|---|
| * (not according to the invention) | | | | | | |

**[Table 8]**

| Test Example | | | 3-10 | 4-4* | 4-5 | 4-6 |
|---|---|---|---|---|---|---|
| (A) | | Macadamia nut oil fatty acid phytosteryl | 5 | 2 | 2 | 5 |
| (B) | (b1) | Decamethyl tetrasiloxane | 14 | 12 | 12 | 17 |
| | (b2) | Squalane | 6 | 5 | 4 | 6 |
| (C) | | Dimethyl distearyl ammonium modified hectorite | 1.7 | 1.7 | 1.7 | 1.7 |
| | | Polyoxyethylene/methylpolysiloxane copolymer | 0.5 | 0.5 | 0.5 | 0.5 |
| (D) | | Ion-exchanged water | 64.3 | 70.3 | 71.3 | 61.3 |
| | | Glycerin | 8 | 8 | 8 | 8 |
| | | Sodium chloride | 0.5 | 0.5 | 0.5 | 0.5 |
| Blending quantity of (A)+(B) | | | 25 | 19 | 18 | 28 |
| Blending quantity of (b1) / Blending quantity of (A)+(B) (%) | | | 56 | 63 | 67 | 61 |
| Evaluation (2): Resilient and supple feeling | | | A | B | A | A |
| Evaluation (3): Stability | | | A* | A* | A* | A* |
| Evaluation (4): Plump feeling | | | A | B | A | A |
| Evaluation (5): Softness | | | A | B | A | A |
| Evaluation (6): Moisturizing effect | | | A | A | A | A |

| | | | | | | |
|---|---|---|---|---|---|---|
| * (not according to the invention) | | | | | | |

According to Table 7 and Table 8, even when the percentage of the (b1) volatile oil having low compatibility with the sterol derivative with respect to component (A) and component (B) satisfies 40 to 85%, the resilient and supple feeling was somewhat poor in Test Examples 4-1 and 4-4 wherein the blending quantity of component (b2) is more than two times of the blending quantity of component (A).

Accordingly, the blending quantity of component (b2) is two times or less of the blending quantity of component (A).

Subsequently, the tests were carried out concerning the emulsion type. The present inventors produced water-in-oil emulsion cosmetics (cream), by the conventional method, with the blending compositions shown in below Table 9, wherein various emulsifying agents were blended. The oil-in-water emulsion cosmetic in the below-described Test Example 5-3 was also produced by the conventional method. Then, each sample was evaluated according to the above-described scoring criteria for evaluation items (2) to (6). The results are shown in Table 9.

**[Table 9]**

| Test Example | | | 5-1* | 5-2* | 5-3* | 5-4* | 5-5* |
|---|---|---|---|---|---|---|---|
| (A) | | Di(phytosteryl/2-octyldodecyl) N-lauroyl- L-glutamate | 5 | 5 | 5 | 5 | 5 |
| (B) | (b1) | Decamethyltetrasiloxane | 20 | 20 | 15 | 15 | 20 |
| (C) | | Dimethyl distearyl ammonium modified hectorite | 1.7 | - | - | - | - |
| | | Polyoxyethylene/methylpolysiloxane copolymer | 0.5 | - | 0.9 | - | - |
| | | Alkyl/polyether modified silicone | - | 2 | - | - | - |
| | | Cross-linked polyether modified silicone/methyl polysiloxane mixture | - | - | 3 | - | - |
| | | Poly(oxyethylene/oxypropylene) methylpolysiloxane copolymer | - | - | - | 1.3 | - |
| | | PEG-60 glyceryl isostearate | - | - | - | - | 1.8 |
| | | Glyceryl monostearate | - | - | - | - | 1.7 |
| | | Propylene glycol monostearate (self-emulsification type) | - | - | - | - | 0.5 |
| (D) | | Ion-exchanged water | 63.8 | 60.5 | 59.6 | 62.2 | 63 |
| | | Glycerin | 8 | 8 | 8 | 8 | 8 |
| | | Sodium chloride | 0.5 | 1 | 1 | 1 | - |
| | | Phenoxyethanol | 0.5 | 0.5 | 0.5 | 0.5 | - |
| | | Ethanol | - | 3 | 7 | 7 | - |
| Blending quantity of (A)+(B) | | | 25 | 25 | 25 | 25 | 20 |
| Blending quantity of (b1) / Blending quantity of (A)+(B) | | | 80 | 80 | 60 | 60 | 20 |
| Emulsion type | | | W/O | W/O | W/O | W/O | O/W |
| Evaluation (2): Resilient and supple feeling | | | A* | A* | A* | A* | C |
| Evaluation (3): Stability | | | A | A | A | A | A |
| Evaluation (4): Plump feeling | | | A | A | B | B | A |
| Evaluation (5): Softness | | | A | A | B | B | A |
| Evaluation (6): Moisturizing effect | | | B | A | B | B | A |

| | | | | | | | |
|---|---|---|---|---|---|---|---|
| * (not according to the invention) | | | | | | | |

According to Test Examples 5-1 to 5-4, various kinds of the (C) emulsifying agents can be used in the water-in-oil emulsion cosmetic of the present invention.

However, the resilient and supple feeling of the sample of Test Example 5-5, which is an oil-in-water emulsion cosmetic, was very poor.

Accordingly, it is necessary that the emulsion cosmetic containing (A) to (D) components is a water-in-oil type emulsion system.

In addition, the present inventors prepared water-in-oil emulsion cosmetics according to the blending compositions shown in below Table 10 to investigate the structure of component (A). Each sample was evaluated, for evaluation items (2) to (6), according to the above-described scoring criteria. The results are shown in Table 10.

**[Table 10]**

| Test Example | | | 6-1* | 6-2 | 6-3* |
|---|---|---|---|---|---|
| (A) | | Di(phytosteryl/2-octyldodecyl) N-lauroyl-L-glutamate | 5 | - | - |
| | | Macadamia nut oil fatty acid phytosteryl | - | 5 | - |
| | | Phytosterol | - | - | 5 |
| (B) | (b1) | Decamethyltetrasiloxane | 20 | 20 | 20 |
| | | Squalane | - | 2 | - |
| (C) | | Dimethyl distearyl ammonium modified hectorite | 1.7 | 1.7 | 1.7 |
| | | Polyoxyethylene/methylpolysiloxane copolymer | 0.5 | 0.5 | 0.5 |
| (D) | | Ion-exchanged water | 64.3 | 62.3 | 64.3 |
| | | Glycerin | 8 | 8 | 8 |
| | | Sodium chloride | 0.5 | 0.5 | 0.5 |
| Evaluation (2): Resilient and supple feeling | | | A* | A* | C |
| Evaluation (3): Stability | | | A | A | A |
| Evaluation (4): Plump feeling | | | C | A | C |
| Evaluation (5): Softness | | | A | A | C |
| Evaluation (6): Moisturizing effect | | | B | B | B |

| | | | | | |
|---|---|---|---|---|---|
| * (not according to the invention) | | | | | |

As shown in Table 10, component (A) in all the test examples has a sterol derivative skeleton. In Test Examples 6-1 and 6-2, wherein component (A) is an ester compound, the resilient and supple feeling was recognized. However, In Test Example 6-3 wherein phytosterol was used, the improvement in the resilient and supple feeling was not recognized. Accordingly, component (A) or the sterol derivative as used in the present invention is an ester compound having a sterol skeleton.

Hereinafter, the formulation examples of the water-in-oil emulsion cosmetics of the present invention will be listed; however, the present invention is not limited by the formulation examples.

### < Formulation Example 1 Cream >

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Di(octyldodecyl/phytosteryl/behenyl) lauroyl glutamate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 2 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Myristoyl methyl-β-alanine (phytosteryl/decyltetradecyl) | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 3 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) (Phytosteryl/behenyl) dimer dilinoleate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 4 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) (Phytosteryl/isostearyl/cetyl/stearyl/behenyl) dimer dilinoleate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 5 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Bis(behenyl/isostearyl/phytosteryl) dimer dilinoleyl dimer dilinoleate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 6 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Di(isostearyl/phytosteryl) dimer dilinoleate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 7 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Di(cholesteryl/behenyl/octyldodecyl) lauroyl glutamate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 8 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Di(cholesteryl/octyldodecyl) lauroyl glutamate | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### <Formulation Example 9 Cream>

| | | |
|---|---|---|
| (1) Glycerin | 5 | mass% |
| (2) Sodium chloride | 0.5 | |
| (3) Water | balance | |
| (4) Disteardimonium hectorite | 1.7 | |
| (5) Polyoxyethylene/methylpolysiloxane copolymer | 0.5 | |
| (6) Decamethyltetrasiloxane | 10 | |
| (7) Squalane | 2 | |
| (8) Cetyl ethylhexanoate | 2 | |
| (9) Petrolatum | 0.5 | |
| (10) Macadamia nut oil fatty acid cholesteryl | 2.5 | |

| | | |
|---|---|---|
| Amount of (bl)/Amount of (A) + (B): 58.8% | | |

### (Production method)

With heating, (4) to (10) were mixed, and the oil phase was uniformly dispersed. The water phase of added (1) to (3) was mixed. The heated water phase was gradually added to the oil phase, and uniform dispersion was carried out with a homodisper. Thus, the emulsified particles were prepared and cooled with stirring, and a cream that is a water-in-oil emulsion cosmetic was produced. The obtained W/O type cream had good stability and the usability excellent in the resilient and supple feeling.

### [Explanations of letters or numerals]

- 1: (A) Sterol derivative
- 2: Additional oil
- 3: Skin

## Claims

1. A water-in-oil emulsion cosmetic comprising the following (A) to (D):
(A) 0.5 to 10 mass% of a sterol derivative selected from di(phytosteryl/octyldodecyl) lauroyl glutamate, macadamia nut oil fatty acid phytosteryl, di(octyldodecyl/phytosteryl/behenyl) lauroyl glutamate, myristoyl methyl-β-alanine (phytosteryl/decyltetradecyl), (phytosteryl/behenyl) dimer dilinoleate, (phytosteryl/isostearyl/cetyl/stearyl/behenyl) dimer dilinoleate, bis(behenyl/isostearyl/phytosteryl) dimer dilinoleyl dimer dilinoleate, di(isostearyl/phytosteryl) dimer dilinoleate, di(cholesteryl/behenyl/octyldodecyl) lauroyl glutamate, di(cholesteryl/octyldodecyl) lauroyl glutamate, and macadamia nut oil fatty acid cholesteryl,
(B) an oil comprising (b1) a volatile oil having low compatibility with (A), and (b2) an oil with a viscosity of less than 1000 mPa•s other than (b1), as determined using a BL-type viscometer (12 rpm, rotor No. 2) at 25°C,
wherein the volatile oil having low compatibility with (A) is selected from hexamethylcyclotrisiloxane, octamethylcyclotetrasiloxane, decamethylcyclopentasiloxane, dodecamethylcyclohexasiloxane, hexamethyldisiloxane, octamethyltrisiloxane, decamethyltetrasiloxane, dodecamethylpentasiloxane, tetradecamethylhexasiloxane, hexadecamethylheptasiloxane, methyltris(trimethylsiloxy)silane, tetrakis(trimethylsiloxy)silane, perfluoromethylcyclopentane, perfluorodimethylcyclohexane, methyl perfluorobutyl ether, methyl perfluoroisobutyl ether, ethyl perfluorobutyl ether, and ethyl perfluoroisobutyl ether,
(C) an emulsifying agent, and
(D) 65 to 85 mass% of an aqueous component which is normally usable in cosmetics, such as, in addition to water, moisturizing agents, water-soluble polymers, UV absorbers, sequestering agents, antioxidants and medicinal agents,
wherein the percentage of component (b1) with respect to component (A) and component (B) is 40 to 85%, and wherein the blending quantity of component (b2) is two times or less of the blending quantity of component (A).

## Patentansprüche

1. Kosmetikum in Form einer Wasser-in-Öl-Emulsion, umfassend die nachfolgenden Komponenten (A) bis (D):
(A) 0.5 bis 10 Masse% eines Sterolderivats ausgewählt aus Di(phytosteryl/octyldodecyl)lauroylglutamat, Macadamianussöl-Fettsäurephytosterylester, Di(octyldodecyl/phytosteryl/behenyl)lauroylglutamat, Myristoyl-methyl-β-alanin(phytosteryl/decyltetradecyl)ester, (Phytosteryl/Behenyl)dimerdilinoleat, (Phytosteryl/Isostearyl/Cetyl/Stearyl/Behenyl)dimer-dilinoleat, Bis(behenyl/isostearyl/phytosteryl)dimer-dilinoleyldimerdilinoleat, Di(isostearyl/phytosteryl)dimerdilinoleat, Di(cholesteryl/behenyl/octyldodecyl)lauroylglutamat, Di(cholesteryl/octyldodecyl)lauroylglutamat und Macadamianussöl-Fettsäurecholesterylester,
(B) ein Öl, welches (b1) ein flüchtiges, eine geringe Kompatibilität mit (A) aufweisendes Öl sowie (b2) ein sich von (b1) unterscheidendes Öl mit einer Viskosität von weniger als 1000 mPa•s, wie unter Verwendung eines viskosimeters vom BL-Typ (12 UpM, Rotor Nr. 2) bei 25°C bestimmt, umfasst,
wobei das flüchtige, eine geringe Kompatibilität mit (A) aufweisende Öl aus Hexamethylcyclotrisiloxan, Octamethylcyclotetrasiloxan, Decamethylcyclopentasiloxan, Dodecamethylcyclohexasiloxan, Hexamethyldisiloxan, Octamethyltrisiloxan, Decamethyltetrasiloxan, Dodecamethylpentasiloxan, Tetradecamethylhexasiloxan, Hexadecamethylheptasiloxan, Methyltris(trimethylsiloxy)silan, Tetrakis(trimethylsiloxy)silan, Perfluormethylcyclopentan, Perfluordimethylcyclohexan, Methylperfluorbutylether, Methylperfluorisobutylether, Ethylperfluorbutylether und Ethylperfluorisobutylether ausgewählt ist,
(C) ein Emulgiermittel, und
(D) 65 bis 85 Masse% einer üblicherweise in Kosmetika verwendbaren wässrigen Komponente wie beispielsweise, neben Wasser, feuchtigkeitsspendenden Mitteln, wasserlöslichen Polymeren, UV-Absorbern, Komplexierungsmitteln, Antioxidationsmitteln und Arzneimitteln,
wobei der prozentuale Anteil an Komponente (b1) in Bezug auf Komponente (A) und Komponente (B) 40 bis 85% beträgt, und
wobei die eingemischte Menge an Komponente (b2) das Doppelte oder weniger der eingemischten Menge an Komponente (A) beträgt.

## Revendications

1. Produit cosmétique de type émulsion eau-dans-huile comprenant les composés (A) à (D) suivants :
(A) 0,5 à 10 % en masse d'un dérivé de stérol choisi parmi le glutamate de di(phytostéryl/octyldodécyl) lauroyle, l'acide gras d'huile de noix de macadamia phytostéryle, le glutamate de di(octyldodécyl/phytostéryl/béhényl) lauroyle, le myristoyl méthyl-β-alanine(phytostéryl/décyltétradécyle), le dilinoléate dimère de (phytostéryl/béhényle), le dilinoléate dimère de (phytostéryl/isostéaryl/cétyl/stéaryl/béhényle), le dilinoléate dimère de dilinoléyle dimère de bis(béhényl/isostéaryl/phytostéryle), le dilinoléate dimère de di(isostéaryl/phytostéryle), le glutamate de di(cholestéryl/béhényl/octyldodécyl) lauroyle, le glutamate de di(cholestéryl/octyldodécyl) lauroyle, et l'acide gras d'huile de noix de macadamia cholestéryle,
(B) une huile comprenant (b1) une huile volatile ayant une faible compatibilité avec (A), et (b2) une huile ayant une viscosité de moins de 1 000 mPa • s autre que (b1), telle que déterminée à l'aide d'un viscomètre de type BL (12 tours/min, rotor N° 2) à 25 °C,
dans lequel l'huile volatile ayant une faible compatibilité avec (A) est choisie parmi l'hexaméthylcyclotrisiloxane, l'octaméthylcyclotétrasiloxane, le décaméthylcyclopentasiloxane, le dodécaméthylcyclohexasiloxane, l'hexaméthyldisiloxane, l'octaméthyltrisiloxane, le décaméthyltétrasiloxane, le dodécaméthylpentasiloxane, le tétradécaméthylhexasiloxane, l'hexadécaméthylheptasiloxane, le méthyltris(triméthylsiloxy)silane, le tétrakis(triméthylsiloxy)silane, le perfluorométhylcyclopentane, le perfluorodiméthylcyclohexane, l'éther de méthyle et de perfluorobutyle, l'éther de méthyle et de perfluoroisobutyle, l'éther d'éthyle et de perfluorobutyle, et l'éther d'éthyle et de perfluoroisobutyle,
(C) un agent émulsifiant, et
(D) 65 à 85 % en masse d'un composé aqueux qui est normalement utilisable en cosmétique, tel que, en plus de l'eau, des agents hydratants, des polymères solubles dans l'eau, des absorbeurs UV, des agents séquestrants, des antioxydants et des agents médicinaux,
dans lequel le pourcentage de composé (b1) par rapport au composé (A) et au composé (B) est de 40 à 85 %, et dans lequel la quantité de mélange du composé (b2) est de deux fois la quantité de mélange du composé (A) ou moins.
